# EUROPÄISCHE PATENTSCHRIFT

(11) **EP 1 233 801 B1**
(45) Veröffentlichungstag und Bekanntmachung des Hinweises auf die Patenterteilung: **22.12.2004**
(21) Anmeldenummer: 00952922.3
(22) Anmeldetag: 12.07.2000
(51) Int. Cl.: A61M 5/20, A61M 5/315, A61M 5/31, A61M 5/46

(54) **INJEKTIONSVORRICHTUNG**
INJECTION DEVICE
DISPOSITIF D'INJECTION

(30) Priorität: 24.07.1999 DE 29912965 U
(43) Veröffentlichungstag der Anmeldung: 28.08.2002
(73) Patentinhaber: Schering Aktiengesellschaft, 13353 Berlin (DE)
(72) Erfinder: WEBER, Wilfried, D-72296 Schopfloch (DE)
(74) Vertreter: Frank, Gerhard, Dipl.-Phys.
(86) Internationale Anmeldenummer: PCT/DE2000/002274
(87) Internationale Veröffentlichungsnummer: WO 2001/007105

(56) Entgegenhaltungen:
- EP-A- 0 904 792
- BE-A- 511 524
- DE-U- 9 321 547
- US-A- 3 880 163
- US-A- 5 318 538
- US-A- 5 643 214

## Beschreibung

### Technischer Hintergrund

Die Erfindung betrifft eine Injektionsvorrichtung gemäß dem Oberbegriff des Schutzanspruchs 1.

### Stand der Technik

Aus dem Stand der Technik sind handelsübliche Injektionsvorrichtungen in ihrer einfachsten Form als Kunststoff-Fertigspritze mit Spritzenkörper, Kanüle mit Nadel, Kolben mit Kolbenstange und einem Halteflansch bekannt, die in der Regel eine sachgemäße Handhabung insbesondere dann erfordern, wenn eine subkutane Injektion durchgeführt werden soll, d.h., wenn zunächst die Nadel bis zu einer möglichst genau definierten Position unter der Haut eingestochen werden soll und erst danach das Arzneimittel injiziert werden soll.

Es ist eine Vorrichtung bekannt (US-A-3 880 163), mit der diese zweistufige Arbeitsweise durchgeführt werden kann, wozu Antriebseinrichtungen wie beispielsweise Fedem und mehr oder weniger komplexe Führungseinrichtungen in zwei koaxial angeordneten Gehäusen angeordnet sind, so dass auf Tastendruck nacheinander die gewünschte zweistufige Arbeitsweise (Einstechhub gefolgt von Injektionshub) gewährleistet ist. Diese Vorrichtung ist technisch aufwendig und im Vergleich zur Spritze selbst sehr platzraubend.

### Darstellung der Erfindung

Aufgabe der Erfindung ist es, die eingangs erwähnte zweistufige Arbeitsweise durch konstruktiv einfachere Zusatzvorrichtungen zu erreichen, um die die Spritze selbst erweitert wird.

Eine Zusatzaufgabe besteht darin, dass diese Zusatzeinrichtungen so konzipiert sein sollen, dass eine "Maskierung" der Nadel im Nicht- Gebrauchszustand gewährleistet sein soll, d.h., dass Beschädigungen oder Verletzungen ausgeschlossen bleiben.

Erfindungsgemäß wird diese Aufgabe gemäß dem kennzeichnenden Teil des Schutzanspruchs 1 gelöst.

Der Grundgedanke der Erfindung ist folglich darin zu sehen, dass bei Verwendung einer handelsüblichen Spritze eine Betriebsweise der Injektionsvorrichtung gewählt wird, bei der sichergestellt ist, dass die vom Patienten entweder manuell oder durch Federkraft auf die Kolbenstange aufgebrachte Kraft sukzessiv zunächst in einen Einstechhub zur Erreichung der subkutanen injektionsposition und erst danach in den Injektionshub zur Injektion des Arzneimittels umgesetzt wird.

Die hierfür erforderlichen Zusatzvorrichtungen beinhalten ein Kopplungselement, dessen Aufgabe es ist, während des Einstechhubes eine Relativbewegung zwischen Kolbenstange und Spritzenkörper zu blockieren, und ein Führungselement, in dem die Spritze zusammen mit dem Kopplungselement verschiebbar gehalten ist und das auf seiner Innenseite eine Auslöseeinrichtung aufweist, die derart auf das Kopplungselement einwirkt, dass nach Durchführung des Einstechhubes die Blockierung der Kolbenstange aufgehoben wird.

Beide Zusatzbauteile, Kopplungselement und Führungselement, können in verschiedenartigen konstruktiven Varianten ausgeführt werden, vorzugsweise als Kunststoffteile im Spritzgußverfahren.

Durch wenige Ergänzungsbauteile kann erreicht werden, dass dieser Funktionsmechanismus auch automatisch ablaufen kann.

Vorteilhafte Ausgestaltungen dieses Prinzips mittels dreier Varianten von Kopplungselement und Führungselement sind in weiteren Unteransprüchen enthalten.

### Kurze Beschreibung der Zeichnungen

Diese drei Ausführungsbeispiele werden nun anhand von Zeichnungen näher erläutert, es zeigen:
- Figur 1:: Eine Explosionsdarstellung des ersten Ausführungsbeispiels der erfindungsgemäßen Injektionsvorrichtung,
- Figur 2:: eine perspektivische Darstellung aus zwei Blickwinkeln der Injektionsvorrichtung der Figur 1 im zusammengebauten Zustand in der Sperrposition,
- Figur 3:: Detaildarstellungen des beim Ausführungsbeispiel gemäß Figur 1 und Figur 2 verwendeten Kopplungselementes,
- Figur 4:: zwei um 90° verdrehte Längsschnitte durch die Injektionsvorrichtung in der Sperrposition,
- Figur 5:: drei Längsschnitte in der Ebene der Figur 4B in der Sperrposition, der Freigabeposition und nach dem Injektionshub,
- Figur 6:: ein zweites Ausführungsbeispiel der Injektionsvorrichtung in der Sperrposition in einem Längsschnitt,
- Figur 7:: ein drittes Ausführungsbeispiel der Injektionsvorrichtung in einem ersten Längsschnitt in der Ebene B-B der Figuren 8 und 9,
- Figur 8:: das dritte Ausführungsbeispiel in einem zweiten Längsschnitt in der Ebene A-A,
- Figur 9:: eine Rückansicht des dritten Ausführungsbeispiels,
- Figur 10:: das dritte Ausführungsbeispiel im Längsschnitt entsprechend Figur 7 nach Durchführung des Einstechhubes,
- Figur 11:: das dritte Ausführungsbeispiel im Längsschnitt entsprechend Figur 8 nach Durchführung des Einstechhubes,
- Figur 12:: das dritte Ausführungsbeispiel in einer ersten perspektivischen Ansicht schräg von vorne, und
- Figur 13:: das dritte Ausführungsbeispiel in einer ersten perspektivischen Ansicht schräg von hinten.

### Beschreibung der Ausführungsbeispiele

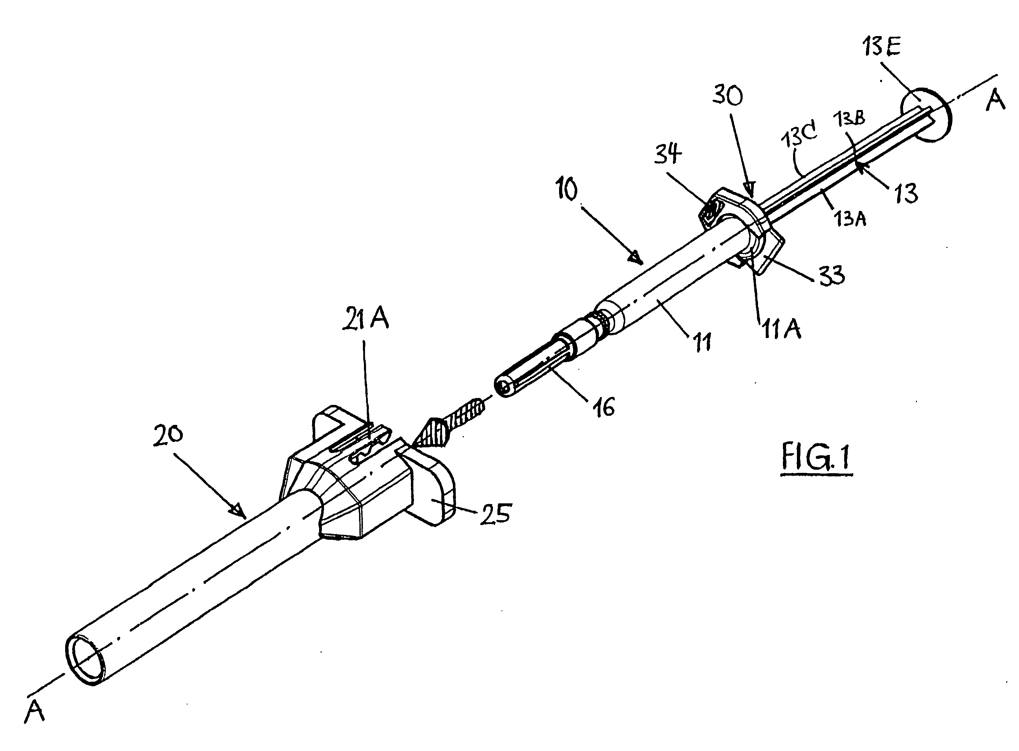

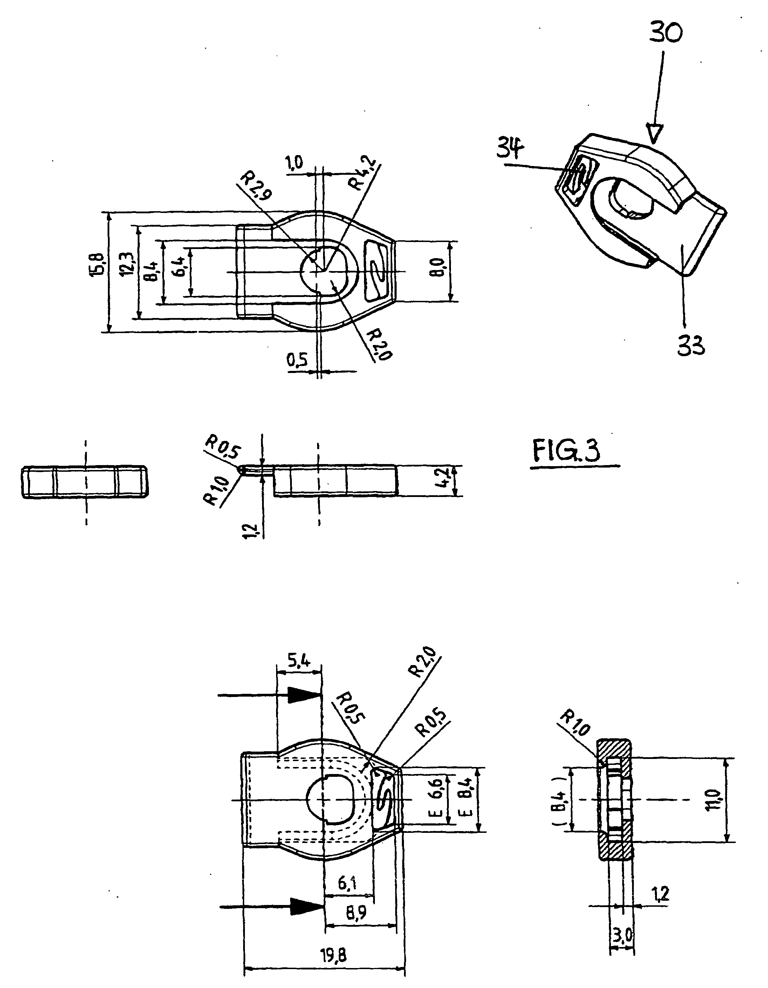

Figur 1 zeigt in der Explosionsdarstellung die drei Komponenten des ersten Ausführungsbeispiels der erfindungsgemäßen Injektionsvorrichtung, nämlich eine Spritze 10 (insbesondere eine handelsübliche Einwegspritze), ein Führungselement 20, zur Aufnahme des Spritzenkörpers 11 und einer Schutzkappe 16 und ein Kopplungselement 30, das eine lösbare Blockierung zwischen dem Flansch 11A des Spritzenkörpers 11 und der Kolbenstange 13 der Spritze 10 herstellt und das ebenfalls im Führungselement 20 untergebracht ist.

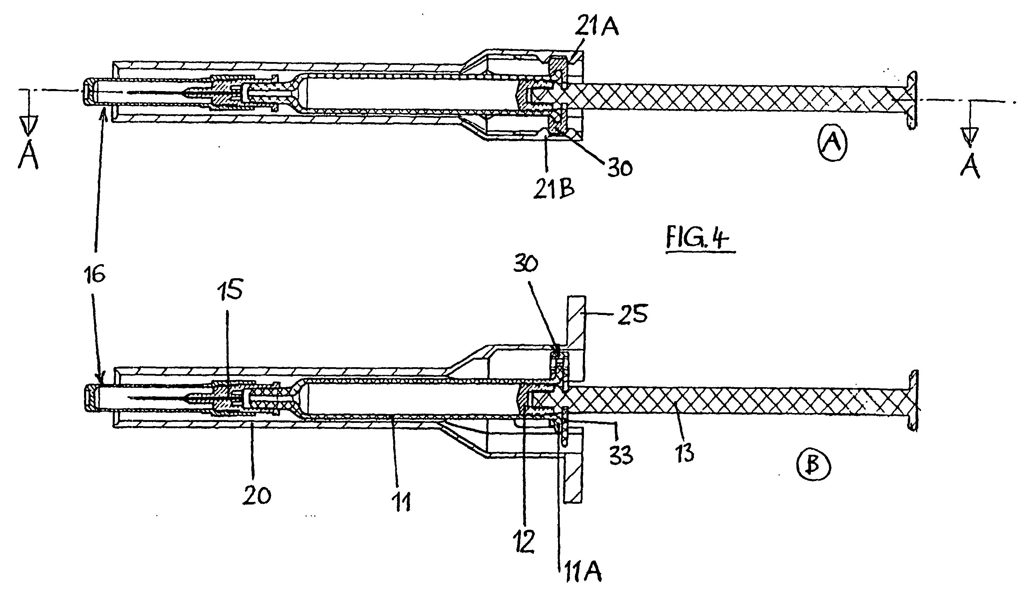

Beim dargestellten ersten Ausführungsbeispiel zeigt Figur 3 den Aufbau des Kopplungselementes 30 aus einem taschenartig geformten Kunststoffelement mit angespritztem Federelement 34, das eine Sperrklinke 33 aufweist, die unter der Vorspannung des Federelementes 34 in der Kolbenstange 13 verrastet und somit die Bewegung des Kolbens (Injektionshub) und damit die Injektion des Arzneimittels blockiert.

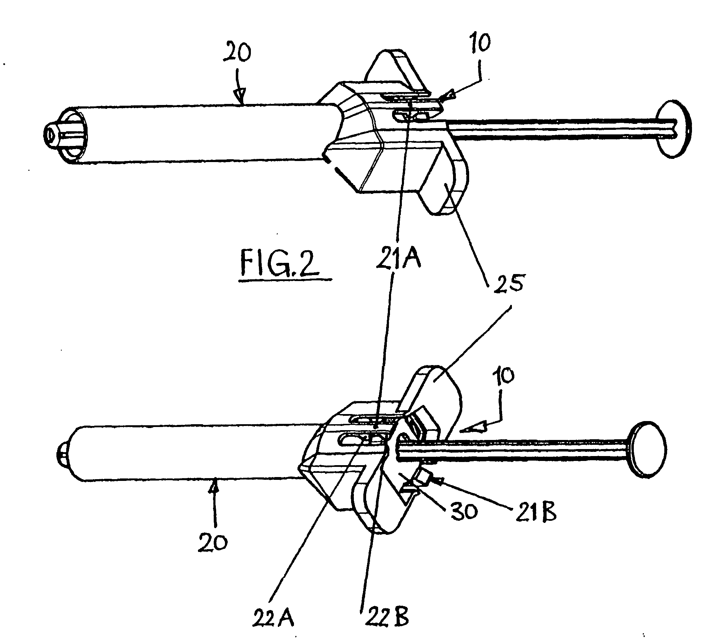

Das Führungselement 20 besteht beim dargestellten ersten Ausführungsbeispiel aus einem röhrenförmigen vorderen Abschnitt, der den vorderen Teil des Spritzenkörpers 11 und die Kanüle mit der Schutzkappe 16 umschließt und sich insbesondere in der in den Figuren 1 und 2 dargestellten Sperrposition über die Nadel der Kanüle hinaus erstreckt, so dass die Nadel "maskiert" ist. Der rückwärtige Teil des Führungselementes 20 ist erweitert und weist einen Aufnahmeraum 24 auf, in dem die Führung des Kopplungselementes 30 über den Einstechhub H1 hinweg erfolgt, und in dem nach Durchlaufen des Einstechhubes H1 eine Auslöseeinrichtung senkrecht zur Injektionsachse A-A auf das Kopplungselement 30 wirkt, derart, dass die Sperrklinke 33 aus ihrer Verrastung mit der Kolbenstange 13 ausgerückt wird, Wenn diese Position erreicht ist, in der dann auch die Nadel aus der vorderen Öffnung des Führungselementes 20 um genau die vorgesehene Einstechtiefe hervorragt, kann der Injektionshub H2 stattfinden , bei dem wie üblich die Kolbenstange 13 mit dem Kolben 12 im Spritzenkörper 11 nach vorne gedrückt wird.

Zum Zusammenwirken mit der Sperrklinke 33 des Kopplungselementes 30 ist die Kolbenstange als Profilteil mit kreuzförmigen Querschnitt ausgebildet. In den vier dadurch gebildeten Längsrippen 13A...13D der Kolbenstange 13 sind geeignete Einschnürungen 13A'...13D' vorgesehen, so dass unabhängig von der Drehposition der Kolbenstange 13 sichergestellt ist, dass die Sperrklinke 33 zumindest in eine dieser Einschnürungen unter der Wirkung des Federelementes 34 einrastet und die Blockierung in der Sperrposition erfolgt. Diese Sperrposition ist in den Figuren 2,4 und 5A dargestellt.

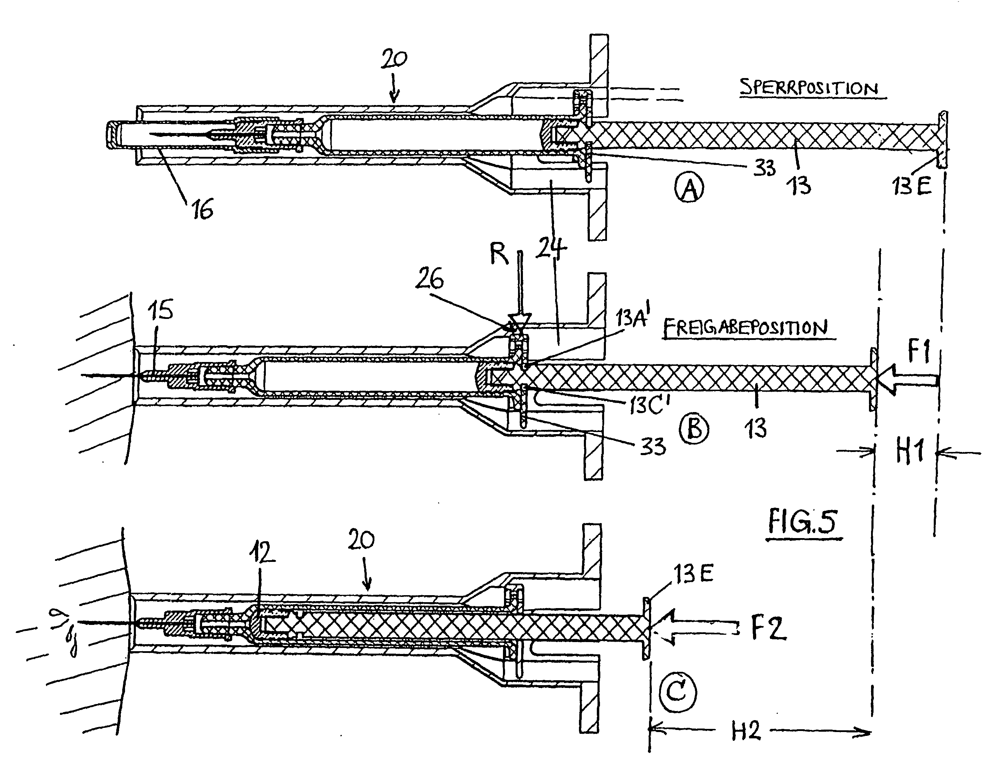

Zur Sicherung der Sperrposition weist der rückwärtige Abschnitt des Führungselementes 20 zwei diametral gegenüberliegende Sperrzungen 21A,21B auf, die mittels Haltenocken 22A,22B bzw. 23A,23B das Kopplungselement 30 in der Sperrposition fixieren, wie dies insbesondere der Figur 2 und der Figur 4A entnehmbar ist.

Im Übergangsbereich zwischen den beiden Abschnitten des Führungselementes 20 ist der Aufnahmeraum 24 kegelstumpfähnlich ausgebildet, wobei insbesondere sein Innenquerschnitt eine Verjüngung 26 aufweist, die nach Art einer schiefen Ebene auf denjenigen Randbereich des Kopplungselementes 30 einwirkt, in dem das Federelement 34 untergebracht ist. Diese Verjüngung 26 stellt also eine sehr einfache Auslöseeinrichtung innerhalb des Führungselementes 20 dar, mit der Wirkung, dass die Blockierung der Kolbenstange 13 aufgehoben wird, wenn der Einstechhub H1 durchgeführt ist.

Die Dimensionierung der Zungen 21A,22A mit ihren zugehörigen Haltenocken 22A,22B bzw. 23A,23B ist dabei so gewählt, dass zur Betätigung der Injektionsvorrichtung, d.h. zur Einleitung des Einstechhubes H1, eine Minimalkraft aufgewendet werden muß; diese Minimalkraft ist zweckmäßigerweise so bemessen, dass eine unbeabsichtigte Betätigung der Injektionsvorrichtung vermieden wird und die Öffnung der Rastzungen so plötzlich erfolgt, dass der Einstechhub H1 unter der Wirkung der Minimalkraft impulsartig abläuft, d.h., dass der Einstechvorgang der Nadel in die Haut sehr schnell und damit sicher und weitgehend schmerzfrei durchgeführt wird.

Zur Handhabung der Injektionsvorrichtung weist das Führungselement 20 einen Betätigungsflansch 25 auf, der funktionell an die Stelle des Flansches 11A des Spritzenkörpers 11 tritt.

Die Vorbereitung und die Durchführung der Injektion wird nun noch kurz beschrieben:

Zunächst wird (in der Regel herstellerseitig) das Kopplungselement 30 seitlich auf den Flansch 11A des Spritzenkörpers 11 aufgesteckt, derart, dass die oben beschriebene Blockierung zwischen Spritzenkörper 11 und Kolbenstange 13 sich einstellt (Figur 1).

Danach wird diese aus Spritze 10 und Kopplungselement 30 bestehende Baueinheit entlang der Injektionsachse A-A in das Führungselement 20 eingeschoben (Pfeil in Figur 1), wobei gegebenenfalls Führungselement 20 und Spritze 10 gegeneinander radial so weit zu verdrehen sind, bis die aufeinander abgestimmten Querschnitte des Aufnahmeraums 24 und des Kopplungselementes 30 die Einführung in den Eingangsbereich des Aufnahmeraums 24 gestatten, bis nach Spreizung der Rastzungen 21A,21B die Gebrauchslage der Injektionsvorrichtung erreicht ist, bei der die Schutzkappe 16 entfernt werden kann, die Nadel der Spritze aber noch innerhalb des rohrförmigen vorderen Abschnittes des Führungselementes 20 gesichert ("maskiert") ist. Diese gebrauchsfertige Position der Injektionsvorrichtung ist in den Figuren 2,4 und 5A dargestellt, so wird in der Regel die Injektionsvorrichtung vom Hersteller an den Kunden bzw. Patienten gelangen , also in der Sperrposition der Injektionsvorrichtung.

Soll nun eine Injektion durchgeführt werden, wird zunächst die Schutzkappe 16 entfemt und die Injektionsvorrichtung wird senkrecht auf die Injektionsstelle aufgesetzt. Wie auch sonst bei der Handhabung von Spritzen üblich, werden Zeige- und Mittelfinger am Betätigungsflansch 25 angelegt und der Daumen auf die Flanschplatte 13E der Kolbenstange 13 aufgedrückt (Figur 5A).Altemativ ist es jedoch auch möglich, das Führungselement 20 mit der einen Hand gegen die Injektionsstelle zu drücken und mit der anderen Hand die Kraft auf die Flanschplatte 13E aufzubringen.

Erreicht nun die Kraft F1, mit der der Daumen die Flanschplatte 13 E beaufschlagt, den oben erwähnten Minimalwert, so springen die Rastzungen 21A,21B auseinander und der Einstechhub H1 läuft impulsartig ab, d.h., die Nadel der Kanüle 15 sticht in die Haut ein und die Auslaßöffnung der Nadel erreicht in kurzer Zeit die zur Injizierung des Arzneimittels vorgesehene subkutane Position unterhalb der Hautoberfläche (Figur 5B). In dieser Position kontaktiert der Randbereich des Kopplungselementes 30 die als Auslöseeinrichtung fungierende Verengung 26 auf der Innenseite des Aufnahmeraums 24 und die Sperrklinke 33 wird aus mindestens einer der Einschnürungen 13A'...13D' ausgerückt (Pfeil R in Figur 5B).

Damit ist die Blockierung der Kolbenstange 13 aufgehoben und die weitere Einwirkung einer Injektionskraft F2 (Figur 5C) führt nun zur Einführung des Kolbens 12 in den Spritzenkörper und damit zur Injektion des Arzneimittels in den Körper (Injektionshub H2).

Nach erfolgter Injektion wird die Injektionsvorrichtung von der Injektionsstelle abgehoben, die Schutzkappe 16 wird wieder aufgesetzt und die Spritze 10 wird an der Flanschplatte 13E gefaßt und vollständig aus dem Führungselement 20 herausgezogen.

Die wesentlichen Vorteile der erfindungsgemäßen Injektionsvorrichtung liegen somit in der Transportsicherheit (keine ungewollte Betätigung) und in der "Maskierung" der Nadel auch bei abgezogener Schutzkappe 16, solange sich die Injektionsvorrichtung in Sperrposition befindet, so dass die Injektionsvorrichtung fest auf der Injektionsstelle aufgesetzt werden kann, ohne dass bereits ein Einstich erfolgt, damit ist eine genaue Ortsdefinition der Einstichstelle erleichtert. Die Einstechtiefe ist eindeutig vorgebbar durch den Abstand zwischen Auslöseeinrichtung und Arretierungsposition des Kopplungselementes, die Ausbildung der Arretierungsposition mit Hilfe von federnden Rastzungen erbringt den zusätzlichen Vorteil des impulsartigen Einstechens der Nadel.

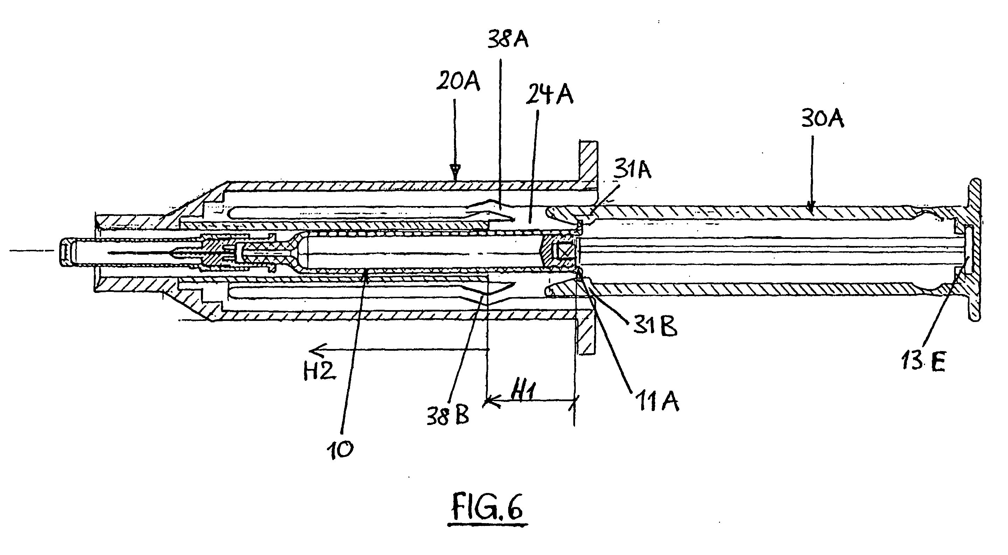

Figur 6 zeigt eine Schnittdarstellung durch ein zweites Ausführungsbeispiel einer erfindungsgemäßen Injektionsvorrichtung, bei dem ebenfalls ein dreiteiliger Aufbau vorgesehen ist, nämlich mit Injektionsspritze 10, Führungselement 20A und Kopplungselement 30A, wobei die letztgenannten beiden Elemente konstruktiv abweichend ausgebildet sind, die prinzipielle Funktion aber entsprechend wie unter dem ersten Ausführungsbeispiel geschildert bleibt, so dass hier lediglich die baulichen Unterschiede kurz erläutert werden sollen:
Das Kopplungselement 30A bewirkt beim zweiten Ausführungsbeispiel eine Kopplung zwischen dem Flansch 11A und der Flanschplatte 13E. Mittels zweier Rastzungen 31A und 31B wird diese formschlüssige Verbindung erreicht.

Der Aufnahmeraum 24A des Führungselementes 20A ist bei dieser Variante als koaxial zur Spritze 10 liegender zylindrischer Hohlraum ausgebildet, in den das Kopplunsgelement 30A im wesentlichen vollständig zur Durchführung von Einstechhub H1 und Injektionshub H2 einführbar ist.

Zur Definition des Übergangs zwischen Einstechhub H1 und Injektionshub H2 zur Lösung der formschlüssigen Kopplung zwischen den Zungen 31A,31B und dem Flansch 11A weist der Aufnahmeraum 24A eine Führungsnut 38A,38B für die beiden Haltezungen 31A,31B auf, die den Haltezungen eine Bewegungskomponente quer zur Injektionsachse A-A aufprägt, so dass die Kopplung mit dem Flansch 11A aufgehoben wird. Der Einstechhub H1 geht somit praktisch nahtlos in den Injektionshub H2 über.

Bei diesem Ausführungsbeispiel ist eine Minimalkraft zur Einleitung des Einstechhubes nicht definiert, so dass Geschwindigkeit und damit Dauer des Einstechvorganges vom Benutzer bzw. Patienten vorgebbar ist, was ggf. die Handhabung geringfügig schwieriger macht, bei besonderen Injektionsanwendungen aber wünschenswert sein kann. Falls gewünscht ,können aber auch bei diesem Ausführungsbeispiel Verrastungen vorgesehen werden, die das Aufbringen einer Minimalkraft zur Einleitung des Einstechhubes erforderlich machen.

Ansonsten ist die Arbeitsweise des zweiten Ausführungsbeispiels der erfindungsgemäßen Injektionsvorrichtung funktionsmäßig gleich der des ersten Ausführungsbeispiels, so dass auf eine weitere Beschreibung verzichtet werden kann.

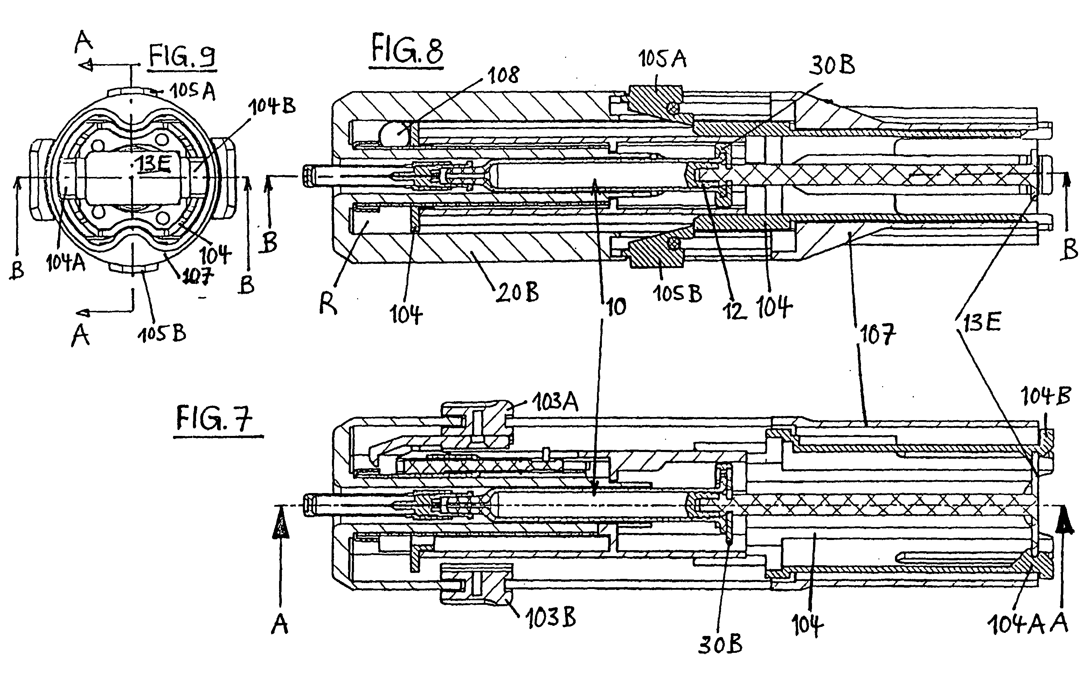

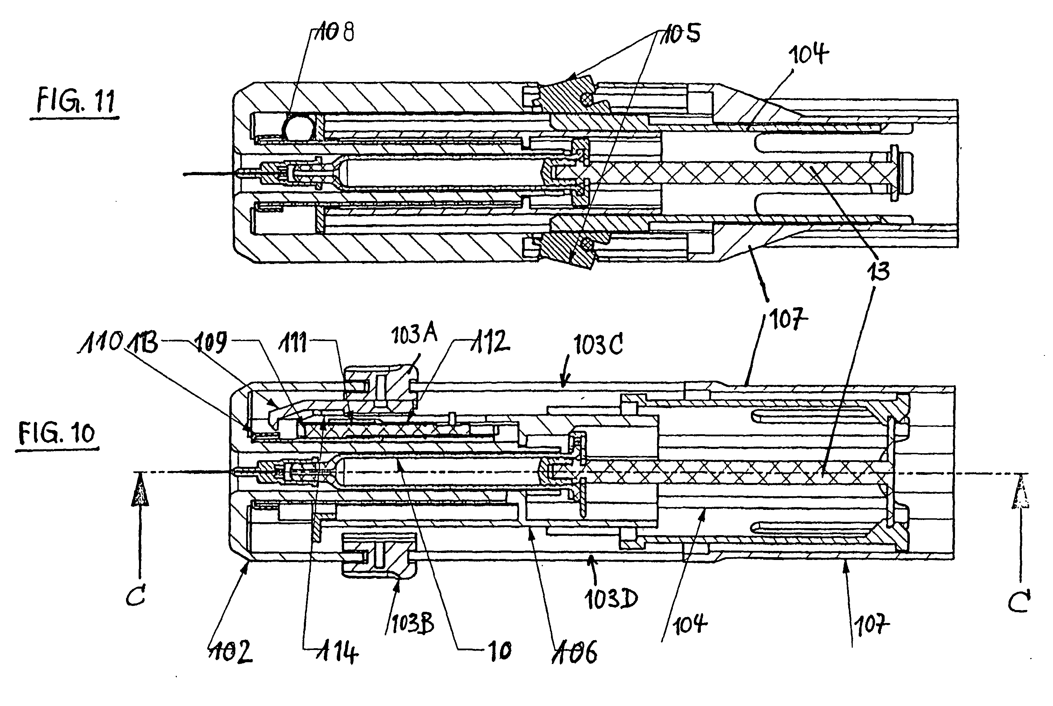

Die Figuren 7 bis 13 zeigen Schnittdarstellungen und perspektivische Darstellungen eines dritten Ausführungsbeispiels der erfindungsgemäßen Injektionsvorrichtung, die sich von den beiden oben beschriebenen Ausführungsbeispielen im wesentlichen dadurch unterscheidet, dass zusätzliche Bauteile vorgesehen sind, die eine automatische Betriebsweise erlauben, d.h., bei der auf das manuelle Eindrücken der Spritze zur Durchführung des Einstechhubes und anschließender Durchführung des Injektionshubes verzichtet werden kann, da dies durch Federkraft erreicht wird.

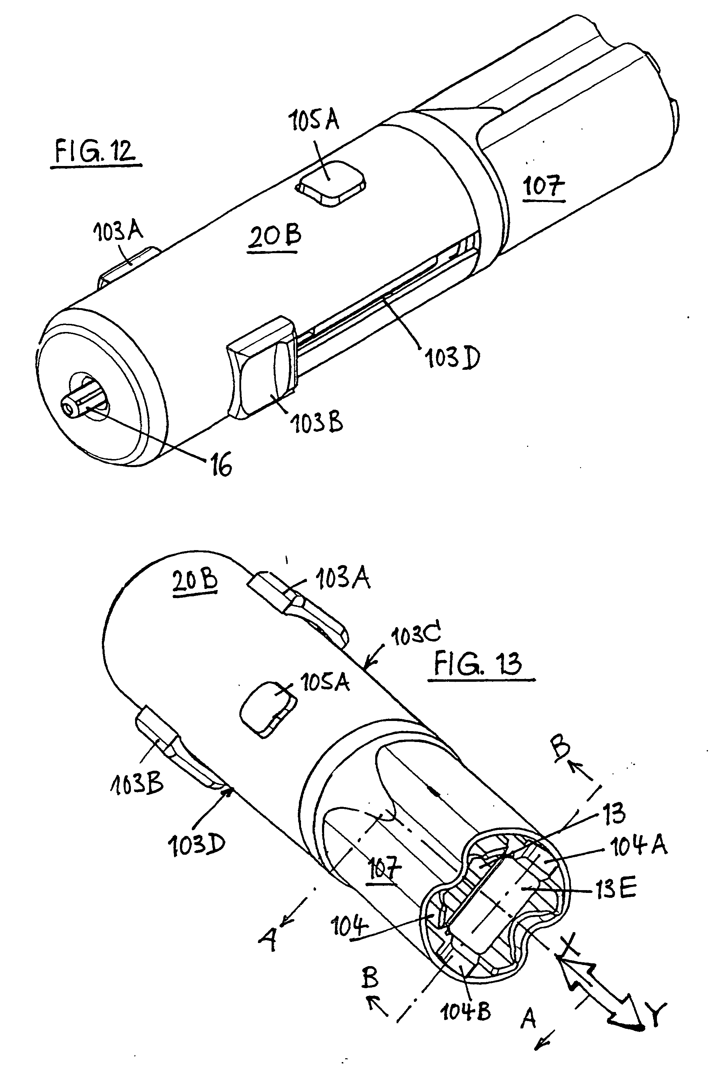

Die wesentliche Aufgabe der Kraftübertragung auf die Spritze wird hierbei von einer Aufnahmehülse 104 übernommen, die ihrerseits in einem Gehäuse 107 axial verschiebbar ist, das eine rückwärtige Fortsetzung eines auch hier vorgesehenen Führungselements 20B ist. Zur Übertragung der Betätigungskraft auf die Spritze 13 weist die Aufnahmehülse 104 zwei zum hinteren Ende der Injektionsvorrichtung zeigende Haltezungen 104A,104B auf, die die Flanschplatte 13E der Kolbenstange 13 der Spritze 10 umgreifen können. Ausserdem ist die Aufnahmehülse 104 mittels einer Rollfeder 108 in Richtung X (Figur 13) vorgespannt, so dass unter der Zusammenwirkung der Rollfeder 108 und der Haltezungen 104A,104B die Spritze 10 in Injektionsrichtung vorgeschoben wird, sofern dies nicht durch geeignete Sperrbauteile verhindert wird. Zur Sperrung bzw. Freigabe dieser Axialbewegung der Aufnahmehülse 104 unter der Wirkung der Rollfeder 108 dient eine Auslöseeinrichtung 105, die zwei diametral gegenüberliegende, aus dem Führungselement 20B ragende Betätigungstasten 105A,105B aufweist. Diese Betätigungstasten 105A,105B weisen je eine Sperrzunge auf, deren Stirnseite sich gegen die Stirnseite der Aufnahmehülse 104 abstützt, wie dies insbesondere aus Figur 8 ersichtlich ist. Die Betätigungstasten 105A,105B sind hierbei um einen Zapfen oder eine entsprechende Achse senkrecht zur Zeichenebene der Figur 8 bzw. Figur 11 schwenkbar gelagert.

Im vorderen Innenbereich des Führungselements 20B ist ein Stößel 109 parallel zur Injektionsachse verschiebbar gehalten, der einerseits durch eine erste Feder 111 in Injektionsrichtung vorgespannt ist, andererseits mit einer zweiten Feder 112 verbunden ist, die gegen die erste Feder 111 wirkt. Frontal vor diesem Stößel 109 befindet sich eine Glocke 110.

Zur Entnahme der Spritze 10 nach erfolgter Injektion ist eine Auswerfereinrichtung 103 vorgesehen, die eine Mitnehmerzunge 113 aufweist, die mit einer der axial verschiebbaren Auswerfergriffen 103A,103B verbunden ist, hierzu sind zwei Axialschlitze 103C,103D im Führungselement 20B vorgesehen.

Die Injektionsvorrichtung arbeitet wie folgt:
Zunächst befindet sich die Aufnahmehülse 104 so weit in ihrer rückwärtigen Position, dass ihre Haltezungen 104A,104B so im Gehäuse 107 positioniert sind, dass sie voneinander weg gespreizt werden können und in ihrer nach innen zeigenden Aufnahmenut der Flansch 13E des Spritzenkolbens 13 eingelegt werden kann. Ist dieser Flansch 13E fixiert ( Figur 13 ), kann die Betätigung der Injektionsvorrichtung erfolgen. Wie bei den ersten beiden Ausführungsbeispielen bewirkt das Kopplungselement 30B in diesem Zustand die feste Kopplung mit der Kolbenstange 13, so dass der Kolben 12 der Spritze 10 nicht verschoben werden kann.

In dieser Bereitschaftsposition verhindern die Sperrklinken der Betätigungstasten 105A, 105B durch ihr Anliegen an der injektionsseitigen Ringschulter der Aufnahmehülse 104 eine Weiterbewegung der Aufnahmehülse 104 in Injektionsrichtung unter der Zugkraft der Rollfeder 108.

Zur Injektion kann nun die Schutzkappe 16 abgenommen werden und die Injektionsvorrichtung auf die Injektionsstelle aufgesetzt werden. Werden dann die beiden Betätigungstasten 105A,105B gleichzeitig gedrückt, so rückt deren Sperrklinke aus dem axialen Weg der Aufnahmehülse 104, so dass diese unter der Wirkung der Rollfeder 108 nach vorne geschoben wird und zunächst den Einstechhub ausführt. Gegen Ende des Einstechhubes wird auch bei diesem Ausführungsbeispiel die Kopplung zwischen dem Kopplungselement 30B und der Kolbenstange 13 gelöst, so dass unter weiterer Axialverschiebung der Aufnahmehülse 104 nunmehr der Injektionshub stattfindet, bei dem der Kolben 12 in der Spritze 10 die Injektionsflüssigkeit in die Hautstelle eindrückt.

Gegen Ende des Injektionshubes wird der durch die erste Feder 111 vorgespannte Stößel 109 aus seiner Raststellung gelöst und schlägt gegen die Glocke 110, wodurch ein akustisches Signal erzeugt wird. Die zweite Feder 112 bewirkt, dass der Stößel 111 sofort nach dem Aufschlagen auf die Glocke 110 wieder von dieser abhebt, damit die Glocke 110 frei schwingen kann und ein deutlich vemehmbares akustisches Signal abgibt.

Nach erfolgter Injektion wird die Injektionsvorrichtung von der Injektionsstelle abgehoben und die Schutzkappe 16 wird wieder aufgesetzt. Die Auswerfergriffe 103A,103B werden in ihren Schlitzen 103C,103D nach hinten gezogen (Richtung Y, Figur 13), so dass der Mitnehmer 113 den Stößel 111 wieder in seine Ausgangs-Rastlage zurück bringt und die Aufnahmehülse 104 gegen die Kraft der Rollfeder 108 wieder in ihre Ausgangslage zurückgeschoben wird, bis die Betätigungstasten 105A,105B wieder vor der stimseitigen Ringschulter der Aufnahmehülse 104 einrasten. Damit ist dann wieder die in den Figuren 7 bis 8 dargestellte Position erreicht, in der die Haltezungen 104A,104B der Aufnahmehülse 104 vom Gehäuse 107 so weit frei gegeben sind, dass die Flanschplatte 13E durch Aufdrücken dieser Haltezungen aus ihrer Verrastung gelöst werden kann und die Spritze axial in Richtung Y aus der Injektionsvorrichtung entnommen werden kann.

## Patentansprüche

1. Injektionsvorrichtung mit einer Spritze mit Spritzenkörper, Kanüle mit Nadel, Kolben mit Kolbenstange und einem die Öffnung des Spritzenkörpers zur Kolbenstange umschließenden Flansch,
**dadurch gekennzeichnet, dass** am Spritzenkörper (11) oder an der Kolbenstange (13) ein Kopplungselement (30,30A,30B) vorgesehen ist, das das Einschieben der Kolbenstange (13) blockiert (Sperrposition), dass die Spritze (10) mit dem Kopplungselement (30,30A,30B) in einem Führungselement (20,20A,20B) um einen Einstechhub (H1) verschiebbar gehalten ist, dass sich das Führungselement (20,20A,20B) nach dem Aufsetzen auf die Injektionsstelle vor Beginn des Einstechhubs (H1) in der Sperrposition bis über die Kanüle (15) hinaus erstreckt, und dass innerhalb des Führungselements (20,20A,20B) eine Auslöseeinrichtung positioniert ist, die die Blockierung der Kolbenstange (13) zur Freigabe des Injektionshubes (H2) löst (Freigabeposition), wenn die Kanüle (15) nach Beendigung des Einstechhubs (H1) ihre subkutane Injektionsposition erreicht hat.

2. Injektionsvorrichtung nach Anspruch 1, **dadurch gekennzeichnet, dass** das Kopplungselement (30) ein am Flansch (11A) gehaltenes Kunststofformteil mit einer Sperrklinke (33) ist, das quer zur Injektionsachse (A-A) derart verschiebbar ist, dass die Sperrklinke (33) in der Sperrposition in die Kolbenstange (13) eingreift und in der Freigabeposition die Kolbenstange (13) freigibt.

3. Injektionsvorrichtung nach Anspruch 2, **dadurch gekennzeichnet, dass** die Kolbenstange (13) mindestens eine in beliebiger Winkelstellung wirksame Rastnase zum formschlüssigen Eingriff der Sperrklinke (33) aufweist.

4. Injektionsvorrichtung nach Anspruch 3, **dadurch gekennzeichnet, dass** die Kolbenstange (13) aus einem Profilteil mit kreuzförmigem Querschnitt gebildet ist, in dessen vier Längsrippen (13A...13D) Einschnürungen (13A'...13D') eingebracht sind, die die Rastnase bilden.

5. Injektionsvorrichtung nach Anspruch 2, **dadurch gekennzeichnet, dass** die Sperrklinke (33) mittels eines Federelementes (34) in seiner Sperrposition im Eingriff mit zumindest einer der Einschnürungen (13A'...13D') der Kolbenstange (13) gehatten ist.

6. Injektionsvorrichtung nach Anspruch 1, **dadurch gekennzeichnet, dass** die Auslöseeinrichtung mindestens eine im Verschiebeweg des Kopplungselements (30A,30B) entlang der Injektionsachse (A-A) angeordnete Steuemase ist.

7. Injektionsvorrichtung nach Anspruch 1, **dadurch gekennzeichnet, dass** das Führungselement (20,20A) ein den Spritzenkörper (11) im wesentlichen konzentrisch umschließendes Gehäuse ist.

8. Injektionsvorrichtung nach Anspruch 7, **dadurch gekennzeichnet, dass** das Gehäuse einen Aufnahmeraum (24) aufweist, der Rastzungen (21A,21B) besitzt, die mittels Haltenocken (22A,22B;23A,23B) das die Sperrklinke (33) taschenartig umschließende Kopplungselement (30) randseitig festhalten.

9. Injektionsvorrichtung nach Anspruch 7, **dadurch gekennzeichnet, dass** der Eintrittsquerschnitt des Aufnahmeraums (24) und der Querschnitt des Kopplungselements (30) so miteinander korrelierend ausgebildet sind, dass nur eine Einschubposition möglich ist.

10. Injektionsvorrichtung nach Anspruch 8, **dadurch gekennzeichnet, dass** das Gehäuse einen um den Eintrittsquerschnitt des Aufnahmeraums (24) verlaufenden Betätigungsflansch (25) aufweist.

11. Injektionsvorrichtung nach Anspruch 2, **dadurch gekennzeichnet, dass** die Auslöseeinrichtung eine kegelstumpfartige Verjüngung (26) des Innenquerschnitts des Aufnahmeraums (24) ist, die die Sperrklinke (33) von der Sperrposition in die Freigabeposition verschiebt.

12. Injektionsvorrichtung nach Anspruch 1, **dadurch gekennzeichnet, dass** das Kopplungselement (30A) aus einem Kunststofformteil gebildet ist, das die stimseitige Flanschplatte (13E) der Kolbenstange (13) der Spritze (10) lösbar mit dem Flansch (11A) des Spritzenkörpers verbindet, und dass der Aufnahmeraum (24A) des Führungselements (20A) mittels der Auslöseeinrichtungen derart ausgebildet ist, dass die Verbindung zwischen Kopplungselement (30A) und Flansch (11A) gelöst wird, wenn der Einstechhub (H1) durchgeführt ist.

13. Injektionsvorrichtung nach Anspruch 12, **dadurch gekennzeichnet, dass** das Kopplungselement (30A) mindestens zwei Haltezungew (31A,31B) aufweist, die den Flansch (11A) rastend umschließen.

14. Injektionsvorrichtung nach Anspruch 13, **dadurch gekennzeichnet, dass** der Aufnahmeraum (24A) mindestens eine Führungsnut (38A,38B) für die Haltezungen (31A,31B) aufweist, die nach Durchlaufen des Einstechhubes (H1) die Haltezungen (31A,31B) auseinanderspreizt und damit den Injektionshub (H2) freigibt.

15. Injektionsvorrichtung nach Anspruch 1, **dadurch gekennzeichnet, dass** zur Verschiebung der Spritze (10) eine innerhalb des Führungselements (20B) verschieb-. bare Aufnahmehülse (104) vorgesehen ist, die die Spritze (10) festhält, von einer Feder (108) in Injektionsrichtung vorgespannt ist und mittels einer Betätigungsvorrichtung (105) aktivierbar ist.

16. Injektionsvorrichtung nach Anspruch 15, **dadurch gekennzeichnet, dass** eine Auswerfereinrichtung (103) vorgesehen ist, die die Aufnahmehülse (104) nach erfolgtem injektionshub wieder gegen die Kraft der Feder (108) zurückschiebt, bis die Betätigungsvorrichtung (105) wieder eingreift.

17. Injektionsvorrichtung nach Anspruch 15, **dadurch gekennzeichnet, dass** eine Signaleinrichtung vorgesehen ist, die von der Aufnahmehülse (104) gegen Ende des Injektionshubes betätigt wird.

18. Injektionsvorrichtung nach Anspruch 15, **dadurch gekennzeichnet, dass** die Aufnahmehülse (104) mindestens zwei die Flanschplatte (13E) umgreifende Haltezungen (104A,104B) aufweist, die in einem Gehäuse (107) derart geführt sind, dass die Umgreifung der Flanschplatte (13E) nur lösbar und die Spritze (10) einlegbar oder entnehmbar ist, wenn die Aufnahmehülse (104) sich in ihrer rückwärtigen Endposition befindet.

## Claims

1. Injection device having a syringe with syringe body, needle assembly with needle, plunger with plunger rod and a flange, which surrounds the opening of the syringe body to the plunger rod, **characterised in that** a coupling element (30, 30A, 30B) is provided on the syringe body (11) or on the plunger rod (13), which coupling element (30, 30A, 30B) blocks the insertion of the plunger rod (13) (locking position), **in that** the syringe (10) is retained with the coupling element (30, 30A, 30B) in a guide element (20, 20A, 20B) so as to be displaceable by a insertion stroke (H1), **in that** the guide element (20, 20A, 20B) after being placed onto the injection site before the beginning of the insertion stroke (H1) in the locked position extends beyond the needle assembly (15), and **in that** inside the guide element (20, 20A, 20B) there is positioned a releasing device, which frees (releasing position) the blocking of the plunger rod (13) for releasing the injection stroke (H2), when the needle assembly (15) has achieved its subcutaneous injection position after completion of the insertion stroke (H1).

2. Injection device according to claim 1, **characterised in that** the coupling element (30) is a plastics material moulded part with a locking pawl (33), said plastics material moulded part being retained on the flange (11A) and being displaceable at right angles to the axis of injection (A-A) in such a manner that the locking pawl (33) engages into the plunger rod (13) in the locking position and releases the plunger rod in the releasing position.

3. Injection device according to claim 2, **characterised in that** the plunger rod (13) has at least one locking projection, which is effective at an arbitrary angular position, for the form-locking engagement of the locking pawl (33).

4. Injection device according to claim 3, **characterised in that** the plunger rod (13) is formed from a profile part with a cross-shaped cross-section, constrictions (13A'...13D'), which form the locking projection, being provided in the four longitudinal ribs (13A...13D) of said profile part.

5. Injection device according to claim 2, **characterised in that** the locking pawl (33) is retained by means of a spring element (34) in its locking position in engagement with at least one of the constrictions (13A'...13D') of the plunger rod (13).

6. Injection device according to claim 1, **characterised in that** the releasing device is at least one control projection, which is disposed in the path of displacement of the coupling element (30A, 30B) along the axis of injection (A-A).

7. Injection device according to claim 1, **characterised in that** the guide element (20, 20A) is a housing which surrounds the syringe body (11) in a substantially concentric manner.

8. Injection device according to claim 7, **characterised in that** the housing has a receiving chamber (24), which has locking tongues (21A, 21B), which, by means of retaining cams (22A, 22B; 23A, 23B), secure the coupling element (30) at its edge, which coupling element (30) surrounds the locking pawl (33) in a pocket-like manner.

9. Injection device according to claim 7, **characterised in that** the inlet cross section of the receiving chamber (24) and the cross-section of the coupling element (30) are correlated to each other in such a manner that only one insertion position is possible.

10. Injection device according to claim 8, **characterised in that** the housing has an actuating flange (25), which extends around the inlet cross-section of the receiving chamber (24).

11. Injection device according to claim 2, **characterised in that** the releasing device is a truncated cone-like tapering (26) of the inner cross-section of the receiving chamber (24), which displaces the locking pawl (33) from the locking position into the releasing position.

12. Injection device according to claim 1, **characterised in that** the coupling element (30A) is formed by a plastics material moulded part, which connects the front flange plate (13E) of the plunger rod (13) of the syringe (10) in a detachable manner to the flange (11A) of the syringe body, and **in that** the receiving chamber (24A) of the guide element (20A) is configured by means of the releasing devices in such a manner that the connection between coupling element (30A) and flange (11A) is released when the insertion stroke (H1) is carried out.

13. Injection device according to claim 12, **characterised in that** the coupling element (30A) has at least two retaining tongues (31A, 31B), which surround the flange (11A) in a locking manner.

14. Injection device according to claim 13, **characterised in that** the receiving chamber (24A) has at least one guide groove (38A, 38B) for the retaining tongues (31A, 31B), which forces apart the retaining tongues (31A, 31B) after the insertion stroke (H1) has been executed and consequently releases the injection stroke (H2).

15. Injection device according to claim 1, **characterised in that**, for displacing the syringe (10), there is a displaceable receiving sleeve (104) provided inside the guide element (20B), which receiving sleeve (104) retains the syringe (10), is pre-tensioned by a spring (108) in the direction of injection and is actuatable by means of an actuating device (105).

16. Injection device according to claim 15, **characterised in that** there is provided an ejection device (103), which, after a successful injection stroke, displaces the receiving sleeve (104) back again in opposition to the force of the spring (108), until the actuating device (105) re-engages.

17. Injection device according to claim 15, **characterised in that** there is provided a signalling device, which is actuated by the receiving sleeve (104) towards the end of the injection stroke.

18. Injection device according to claim 15, **characterised in that** the receiving sleeve (104) has at least two retaining tongues (104A, 104B), which embrace the flange plate (13E) and are guided in a housing (107) in such a manner that the embracing of the flange plate (13E) is detachable and the syringe (10) is insertable or removable only when the receiving sleeve (104) is situated in its rearward end position.

## Revendications

1. Dispositif d'injection comprenant une seringue avec corps de seringue, canule avec aiguille, piston avec tige de piston et collerette entourant l'ouverture du corps de piston vers la tige de piston,
**caractérisé en ce qu'**il est prévu sur le corps de seringue (11) ou sur la tige de piston (13) un élément de couplage (30, 30A, 30B) qui bloque l'enfoncement de la tige de piston (13) (position de blocage), **en ce que** la seringue (10) est montée coulissante avec l'élément de couplage (30, 30A, 30B) dans un élément de guidage (20, 20A, 20B) sur une course de piqûre (H1), **en ce que** dans la position de blocage, après application sur le site d'injection et avant le début de la course de piqûre (H1), l'élément de guidage (20, 20A, 20B) s'étend jusqu'au-delà de la canule (15), et **en ce qu'**à l'intérieur de l'élément de guidage (20, 20A, 20B) est positionné un dispositif de déclenchement, qui libère (position de libération) le blocage de la tige de piston (13) pour permettre la course d'injection (H2), lorsque la canule (15) a atteint sa position sous-cutanée d'injection après achèvement de la course de piqûre (H1).

2. Dispositif d'injection selon la revendication 1, **caractérisé en ce que** l'élément de couplage (30) est une pièce moulée en matière plastique, munie d'un cliquet de blocage (33) et montée sur la collerette (11A), cette pièce moulée étant déplaçable transversalement à l'axe d'injection (A-A) de façon que le cliquet de blocage (33) en position de blocage vient en prise dans la tige de piston (13) et en position de libération libère la tige de piston (13).

3. Dispositif d'injection selon la revendication 2, **caractérisé en ce que** la tige de piston (13) présente au moins un bec d'encliquetage opérationnel dans une position angulaire quelconque pour que le cliquet de blocage (33) vienne en prise par formes conjuguées.

4. Dispositif d'injection selon la revendication 3, **caractérisé en ce que** la tige de piston (13) est constituée d'une pièce profilée à section transversale cruciforme dans les quatre nervures longitudinales (13A...13D) de laquelle sont logés des rétrécissements (13A'...13D') qui forment les becs d'encliquetage.

5. Dispositif d'injection selon la revendication 2, **caractérisé en ce que** le cliquet de blocage (33) est maintenu dans sa position de blocage en prise avec.au moins l'un des rétrécissements (13A'...13D') de la tige de piston (13) au moyen d'un élément élastique (34).

6. Dispositif d'injection selon la revendication 1, **caractérisé en ce que** le dispositif de déclenchement est au moins un bec de commande agencé dans le trajet de coulissement de l'élément de couplage (30A, 30B) le long de l'axe d'injection (A-A).

7. Dispositif d'injection selon la revendication 1, **caractérisé en ce que** l'élément de guidage (20, 20A) est un boîtier entourant de manière sensiblement concentrique le corps de seringue (11).

8. Dispositif d'injection selon la revendication 7, **caractérisé en ce que** le boîtier présente une chambre de réception (24) qui possède des languettes d'encliquetage (21A, 21B) qui au moyen de bossages de retenue (22A, 22B ; 23A, 23B) fixent du côté du bord l'élément de couplage (30) enveloppant le cliquet de blocage (33) à la manière d'une poche.

9. Dispositif d'injection selon la revendication 7, **caractérisé en ce que** la section transversale d'entrée de la chambre de réception (24) et la section transversale de l'élément de couplage (30) sont configurées de manière mutuellement corrélée de sorte qu'une seule position d'introduction est possible,

10. Dispositif d'injection selon la revendication 8, **caractérisé en ce que** le boîtier présente une collerette d'actionnement (25) s'étendant autour de la section transversale d'entrée de la chambre de réception (24).

11. Dispositif d'injection selon la revendication 2, **caractérisé en ce que** le dispositif de déclenchement est un rétrécissement (26) du genre tronconique affectant la section transversale intérieure de la chambre de réception (24), lequel rétrécissement fait passer le cliquet de blocage (33) de la position de blocage à la position de libération.

12. Dispositif d'injection selon la revendication 1, **caractérisé en ce que** l'élément de couplage (30A) est formé d'une pièce moulée en matière plastique, qui relie la plaquette frontale formant collerette (13E) de la tige de piston (13) de la seringue (10) de manière amovible avec la collerette (11A) du corps de seringue, et **en ce que** la chambre de réception (24A) de l'élément de guidage (20A) est formée au moyen des dispositifs de déclenchement de façon que la liaison entre l'élément de couplage (30) et la collerette (11A) est libérée lorsque la course de piqûre (H1) est effectuée.

13. Dispositif d'injection selon la revendication 12, **caractérisé en ce que** l'élément de couplage (30A) présente au moins deux languettes de maintien (31A, 31B) qui entourent la collerette (11A) avec encliquetage.

14. Dispositif d'injection selon la revendication 13, **caractérisé en ce que** la chambre de réception (24A) présente pour les languettes de maintien (31A, 31B) au moins une rainure de guidage (38A, 38B) qui écarte l'une de l'autre les languettes de maintien (31A, 31B) une fois la course de piqûre (H1) effectuée et libère ainsi la course d'injection (H2),

15. Dispositif d'injection selon la revendication 1, **caractérisé en ce qu'**il est prévu pour le coulissement de la seringue (10) un manchon récepteur (104) coulissant à l'intérieur de l'élément de guidage (20B), lequel manchon fixe la seringue (10), est précontraint dans la direction d'injection par un ressort (108) et peut être activé au moyen d'un dispositif d'actionnement (105).

16. Dispositif d'injection selon la revendication 15, **caractérisé en ce qu'**il est prévu un dispositif d'éjection (103), qui repousse le manchon récepteur (104) à l'encontre de la force du ressort (108) une fois effectuée la course d'injection, jusqu'à ce que le dispositif d'actionnement (105) revienne en prise.

17. Dispositif d'injection selon la revendication 15, **caractérisé en ce qu'**il est prévu un dispositif de signalisation qui est actionné par le manchon récepteur (104) vers la fin de la course d'injection.

18. Dispositif d'injection selon la revendication 15, **caractérisé en ce que** le manchon récepteur (104) présente au moins deux languettes de maintien (104A, 104B) qui viennent en prise autour de la plaquette formant collerette (13E), qui sont guidées dans un boîtier (107) de sorte que la prise autour de la plaquette formant collerette (13E) n'est qu'amovible et la seringue (10) peut être mise en place ou enlevée, lorsque le manchon récepteur (104) se trouve dans sa position extrême arrière.
